(19) Europäisches Patentamt
European Patent Office
Office européen des brevets

(11) Publication number: **0 001 873**
**A1**

# (12) EUROPEAN PATENT APPLICATION

(21) Application number: 78300205.8

(22) Date of filing: 26.07.78

(51) Int. Cl.²: C 07 C 11/21
C 07 C 33/02, C 07 C 49/73

(30) Priority: 11.08.77 JP 96765/77

(43) Date of publication of application:
16.05.79 Bulletin 79/10

(84) Designated contracting states:
CH DE FR GB NL

(71) Applicant: Takeda Yakuhin Kogyo Kabushiki Kaisha
27, Doshomachi 2-Chome
Higashi-ku Osaka, (541)(JP)

(72) Inventor: Shinji, Terao
26-3 Shinsenri-minamimachi 2-chome
Toyonaka Osaka, (565)(JP)

(72) Inventor: Mitsuru, Shiraishi
50-1, Yamadaminami
Suita Osaka (565)(JP)

(72) Inventor: Kaneyoshi, Kato
50-1, Yamadaminami
Suita Osaka, (565)(JP)

(74) Representative: Laredo, Jack Joseph et al,
Elkington and Fife High Holborn House 52/54 High Holborn
London, WC1V 6SH(GB)

## (54) Method of producing polyisoprenoid compounds by carbon-carbon bond formation.

(57) A polyisoprenoid compound is prepared by reacting a compound $R^1CH_2Y$ wherein Y is lower alkylsulfonyl or arylsulfonyl, and $R^1$ is isoprenoid or polyisoprenoid, with a $XCH_2R^2$ compound wherein X is halogen and $R^2$ has the same meaning as $R^1$, in a polar solvent and in the presence of sodium hydride, potassium hydride, sodium tertiary-alkoxide, potassium tertiary-alkoxide, sodium amide or potassium amide, to obtain a compound

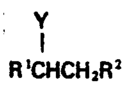

which, in turn, is subjected to a reductive desulfonylation of the group Y, whereby a compound $R^1CH_2CH_2R^2$ is obtained.

EP 0 001 873 A1

Title

Method of C-C Coupling

-------

This invention relates to a commercially advantageous method of carbon-carbon bond formation.

Carbon-carbon bond formation reactions (hereinafter sometimes referred to as C-C coupling) constitute an important class of reactions in the art of organic synthesis, and a variety of such reactions have been hitherto proposed. Thus, it is known that a C-C coupling can be accomplished with respect to a methylene group adjacent to one of -S-, -SO-, $-SO_2-$ and similar groups, by first activating (carbanionizing) the carbon atom of the methylene group with a strongly basic lithium compound such as n-butyllithium, phenyllithium, lithium diethylamide or the like, and thereafter causing an electrophilic agent to act upon the carbanion. However, this type of reaction must usually be conducted in a dry ethereal solvent such as dry tetrahydrofuran or diethyl ether together with a cosolvent such as hexamethylphosphoramide (HMPA), in an inert gaseous atmosphere and at a low temperature of around -70°C. In view of the need to use, as just described, a lithium compound, an anhydrous solvent and a low reaction temperature, for instance, such a method cannot be considered commercially advantageous. In particular, because of the recently suspected carcinogenicity of HMPA, it is desirable to avoid using this solvent as much as possible.

We have now succeeded in the establishment of a novel and commercially profitable method of C-C coupling with respect to isoprenoid derivatives having a methylene group adjacent to an $-SO_2-$ group.

This invention is therefore directed to a method for producing a polyisoprenoid compound by carbon-carbon bond formation characterized in that a sulfonyl compound of the formula:

$$R^1CH_2Y \qquad (IV)$$

wherein Y is lower alkylsulfonyl or arylsulfonyl, and $R^1$ is isoprenoid or polyisoprenoid,

is reacted with a halogeno-compound of the formula:

$$XCH_2R^2 \qquad (III)$$

wherein X is halogen and $R^2$ has the same meaning as $R^1$,

in a polar solvent and in the presence of sodium hydride, potassium hydride, sodium tert.-alkoxide, potassium tert.-alkoxide, sodium amide or potassium amide, to yield a compound of the formula:

$$R^1\underset{}{\overset{Y}{C}}HCH_2R^2 \qquad (II)$$

wherein Y, $R^1$ and $R^2$ have the same meanings as defined above,

which, in turn, is subjected to a reductive desulfonylation of the group Y, whereby a compound of the formula:

$$R^1CH_2CH_2R^2 \qquad (I)$$

wherein $R^1$ and $R^2$ have the same meanings as defined above,

is obtained.

Referring to the above formulae, the lower alkylsulfonyl group Y is preferably a sulfonyl group whose alkyl moiety contains about 1 to 4 carbon atoms (e.g. methyl or ethyl), and the arylsulfonyl group, as represented by Y is preferably benzenesulfonyl, *p*-toluenesulfonyl or the like. Among them, arylsulfonyl is preferably employed. The halogen X is preferably chlorine or bromine.

The isoprenoid or polyisoprenoid group, as designated by $R^1$ or $R^2$, may be for example a group of the following formula

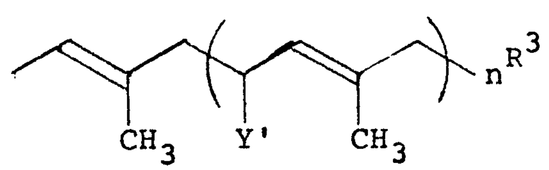

(Va)

or

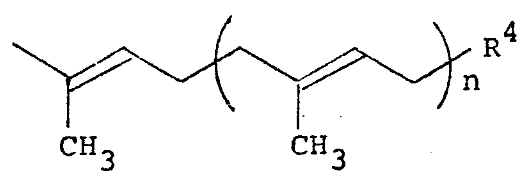

(Vb)

wherein:

$R^3$ is hydrogen or hydroxy which may optionally be protected;

$R^4$ is hydroxy, which may optionally be protected, or a group of the formula:

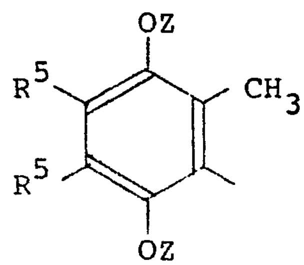

(VI)

(wherein each $R^5$ is methyl or methoxy, or two $R^5$-groups, taken together, represent a group of the formula -CH=CH-CH=CH-. and Z is hydrogen or a protective group) or the corresponding quinone;

Y' is hydrogen or is the same as Y (defined hereinbefore); and

n is an integer 0 to 10 inclusive.

It should be understood that, although in the above formulae (Va) and (Vb) the isoprenoid moieties are shown in the trans-oriented forms for the sake of convenience, they may alternatively be in the cis-oriented form.

The protective group on the protected hydroxy groups $R^3$ and $R^4$ and the protective group designated by Z may both be any one of the protective groups usually employed in the art, provided they are capable of protecting the respective hydroxy groups. Thus, for example, $C_{1-4}$ alkyl (e.g. methyl, ethyl), $C_{1-4}$ alkoxymethyl (e.g. methoxymethyl or ethoxymethyl), aralkyl (e.g. benzyl, p-nitrogenzyl or p-methoxybenzyl), acyl (e.g. alkanoyl groups containing up to 4 carbon atoms such as acetyl or propionyl, benzoyl, p-nitrobenzoyl or phenylacetyl), tetrahydropyranyl and tetrahydrofuranyl may be mentioned. Above all, methoxymethyl, benzyl, acetyl, tetrahydropyranyl and tetrahydrofuranyl are particularly desirable. Where $R^1$ or

$R^2$ in the starting compound (IV) or (III), as the case may be, is a group of the formula (Vb), it is preferable that $R^4$ should be a hydroxy group which may optionally be protected or a group of the formula (VI) where Z is a protective group. Y' is hydrogen or a group represented by Y which has been described hereinbefore, and may occur in an optional combination related to the n-number of isoprenoid units in the formula (Va).

The polar solvent employed in the method of this invention may be for example dimethylformamide, dimethylacetamide, tert-butyl alcohol, tert-amyl alcohol, dimethylsulfoxide, tetrahydrofuran or 1,2-dimethoxyethane. Above all, dimethylformamide, tert-butyl alcohol and tetrahydrofuran are particularly desirable. These solvents may be employed either alone or in a mixture with other solvents.

The aforesaid sodium hydride, potassium hydride, sodium tert-alkoxide, potassium tert-alkoxide, sodium amide or potassium amide (hereinafter sometimes referred to generally as a 'basic reagent') is used for the purpose of converting the methylene carbon of the sulfonyl compound (IV) into a carbanion. As examples of the sodium or potassium tert.-alkoxide, there may be mentioned alkoxides of 4 to 6 carbon atoms such as sodium tert.-butoxide, potassium tert.-butoxide, sodium tert.-amyloxide and potassium tert.-amyloxide. Among the basic reagents, sodium hydride, sodium tert.-butoxide and potassium tert.-butoxide are particularly advantageous.

The method according to this invention is now explained in detail. First, the reaction of a sulfonyl compound (IV) with halogeno-compound (III) may be carried out either by dissolving the compound (IV)

previously in a polar solvent, adding the 'basic reagent' to the solution to produce a carbanion and finally adding (III), or by dissolving both of the compounds (IV) and (III) in a polar solvent and then adding the 'basic reagent'. While the reaction proceeds more or less satisfactorily in the presence of air, it is preferably conducted in an inert gaseous atmosphere (e.g. nitrogen, helium or argon). The molar ratio of (IV) to (III) is normally 1 : 1, although one of the materials for C-C coupling may be used in excess depending on the particular species of compounds employed. The proper amount of the 'basic reagent' depends on the number of Y (sulfonyl) groups occurring in the starting material; normally, it is advantageous to employ about 1 to 2 equivalents per Y group. While the reaction rate depends on the rate of formation of the carbanion, the reaction normally proceeds rapidly and goes to completion within 2 hours in many cases. The reaction may be carried out satisfactorily in the range of from -10° to +30°C and need not particularly be conducted at a very low temperature.

This coupling reaction is never accompanied by an isomerization at the double bond of the isoprenoid-containing compound; in other words, the *cis*- or *trans*-configuration of the starting compound is retained. The coupling product (II) obtained by the above reaction can be isolated by procedures conventional *per se*.

The above coupling product (II) may be isolated beforehand or the reaction mixture containing (II) as such may be directly subjected to the reductive desulfonylation with respect to the lower alkylsulfonyl

or arylsulfonyl group Y. In either case, the contemplated compound (I) can be obtained by this reductive desulfonylation. The said reductive desulfonylation may be conducted by various procedures known per se. Thus, for example, reduction with an amine (e.g. ethylamine, methylamine, diethylamine or ammonia) and an alkali metal (e.g. lithium, sodium or potassium), reduction with sodium amalgam and reduction with Raney's nickel may be mentioned. When there is a reducible group in the substituent $R^1$ or $R^2$ of the compound (II), this reductive desulfonylation not only results in the elimination of Y but may also result in the reduction of such a reducible group. For example, when $R^1$ or $R^2$ of the compound (II) contains a reducible $-CH=CH-CH=CH-$ group as formed by two groups $R^5$ taken together, the elimination of Y may be accompanied by the reduction of this particular reducible group, giving rise to a desired compound containing a $-CH_2-CH=CH-CH_2-$ group. Further, when there is a protective group (e.g. benzyl) which is reductively removable, the elimination of Y may be accompanied by the removal of the protective group.

In the above procedure, when the substituent $R^4$ of the product is the hydroquinone group (VI), it may be converted to the corresponding quinone by a conventional oxidation procedure (e.g. oxidation with ferric chloride, air oxidation or oxidation with silver oxide).

The desired compound (I) produced in the above manner can be isolated from the reaction mixture and purified by conventional separatory-purification procedures (e.g. chromatography or distillation).

The method according to this invention can be applied to the production of polyisoprenoid compounds such as polyisoprenoid hydrocarbons (e.g. squalene), polyprenyl alcohols [such as tetraprenyl alcohol, pentaprenyl alcohol, hexaprenyl alcohol, heptaprenyl alcohol, octaprenyl alcohol, nonaprenyl alcohol (inclusive of solanesol), decaprenyl alcohol, undecaprenyl alcohol, dodecaprenyl alcohol, tridecaprenyl alcohol, hexadecaprenyl alcohol or eicosaprenyl alcohol], ubiquinones (e.g. ubiquinone-7, ubiquinone-8, ubiquinone-9 or ubiquinone-10), menaquinones (e.g. menaquinone-4, menaquinone-5 or menaquinone-9), tocoquinones and other like polyisoprenoid compounds.

The present method has a number of commercially advantageous features. These advantages include the following, among others:

(1) The method involves the use of a 'basic reagent' which is relatively inexpensive and safe, and hence easier to handle than the strongly basic lithium compound hitherto employed.

(2) The reaction can be conducted under icecooling or at temperatures near room temperature (about -10 to +30°C) and need not be carried out at temperatures as low as about -70°C.

(3) The method achieves excellent reaction yields and very high stereo-specificity.

(4) In the present method, it is possible to employ a solvent which is inexpensive, non-inflammable and safe to human beings and unnecessary to employ a strictly dehydrated solvent.

(5) The reaction may be satisfactorily carried out in the ordinary atmosphere and need not necessarily be conducted in an inert gaseous atmosphere.

(6) The method involves the use of readily available starting materials and permits the formation of C-C bonds without affecting the chemically labile functional groups in starting materials.

The starting compounds (IV) and (III) employed in accordance with this invention may be easily produced , for example by the general method set out below, or other known methods, from known compounds [cf. Abstracts of Reports of the 97th Annual Meeting (1977) of the Pharmaceutical Society of Japan, II, 167(6E4-4)]:

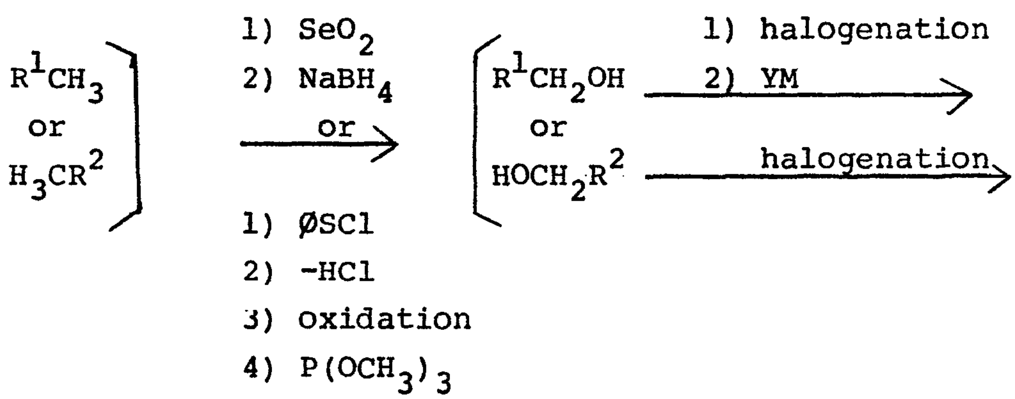


(IV)

(III)

[In the above formulae, $R^1$, $R^2$ and Y have the same meanings as hereinbefore defined; Ø means phenyl; and M means alkali metal].

This invention will hereinafter be further described by way of reference examples and working examples which, however, should not be construed as limiting the scope of the invention.

In those reference and working examples, Ts means p-toluenesulfonyl; Bz means benzyl; Ø means phenyl; Pr means :

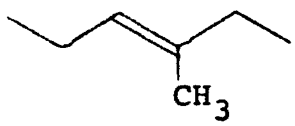 ; Q means: 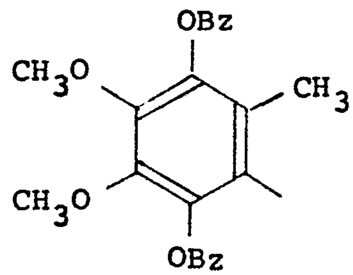 ;

Q' means: 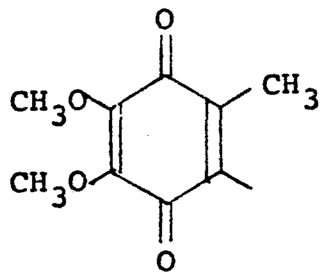 ;

MK means : 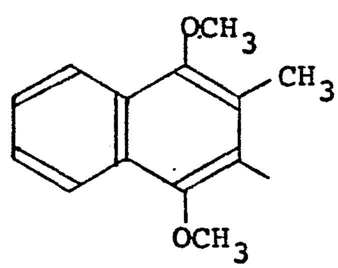 ;

MK' means: 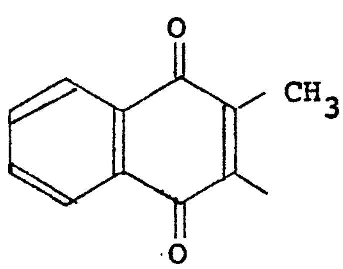 ;

E means: 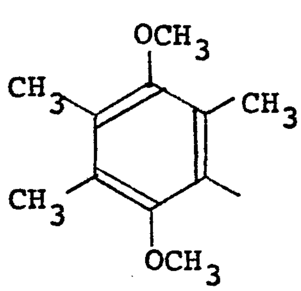 ;

E' means : 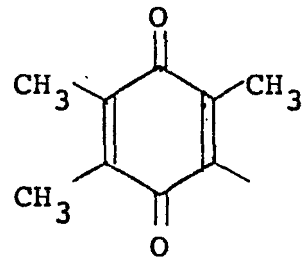 ; THF means tetrahydrofuran; DMF means dimethylformamide; t-BuOH means tert.-butyl alcohol; t-BuONa means sodium tert.-butoxide; t-BuOK means potassium tert.-butoxide; and δ denotes the chemical shift in NMR (nuclear magnetic resonance) spectra as measured in deuteriochlorform, with TMS as the internal standard.

## Example I

### Example I-1

A sulfonyl compound (IV: Ts $(Pr)_9$H, m.p. 50-51°C, 1.54 g, 2 m moles) and a bromide (III: BzO-Pr-Br, 510 mg, 2 m moles) were dissolved in a mixture of THF (16 ml) and DMF (4 ml) and the mixed solution was cooled to -5°C. Under argon gas streams, potassium tert-butoxide (460 mg, 4 m moles) was gradually added.

After the addition had been completed, the mixture was stirred for 20 minutes. The reaction mixture was diluted with water (50 ml) and extracted with isopropyl ether (100 ml). The organic layer was wahsed with water, dried ($Na_2SO_4$) and concentrated under reduced pressure. The product was chromatographed on silica gel and eluted with isopropyl ether-hexane (1:1). By the above procedure there was obtained the coupled compound (II)

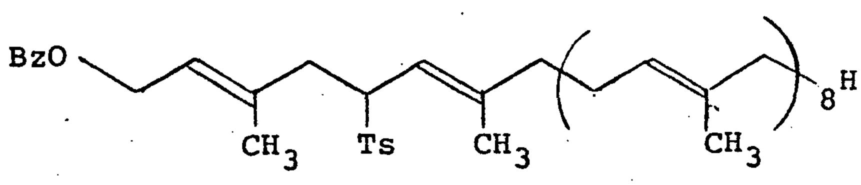

in a yield of 1.736 g. (See Table 1 below).

Example I-5

A sulfonyl compound (IV: Ts $-(Pr)_2$ H, 1.46 g, 5 m moles) and a bromide (III: BzO $-(Pr)_2Br$, 1.62 g) were dissolved in DMF (15 ml) and the mixed solution was cooled to 5°C. Under argon gas streams, sodium hydride (50% oil dispersion, 0.25 g, 5 m moles) was added and stirred for 30 minutes. To this reaction mixture was added a further amount (0.1 g, 2 m moles) of sodium hydride, followed by stirring under the same conditions for 30 minutes. The reaction mixture was diluted with water (40 ml) and extracted with isopropyl ether. The product was isolated and purified by chromatography on silica gel in the conventional manner. By the above procedure there was obtained the desired compound (II):

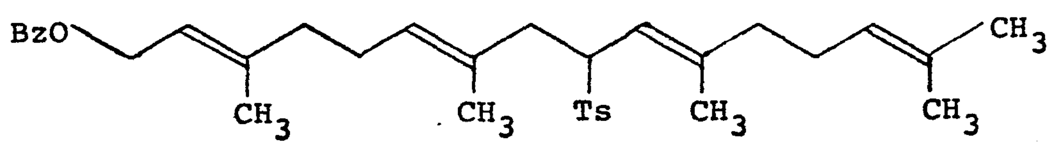
,

in a yield of 2.40 g, 90% (see Table 1 below).

Example I-9

A sulfonyl compound (IV: Ts $-(Pr)_2OH$, 1.54 g, 5 m moles) and bromide (III: BzO $-(Pr)_2Br$, 1.62 g, 5 m moles) were dissolved in DMF (15 ml) and the mixed solution was cooled to 5°C. Then sodium hydride (50% oil dispersion, 0.38 g, 7.5 m moles) was added and, under argon gas streams, the mixture was stirred under the same conditions for 90 minutes. The reaction mixture was diluted with water (40 ml) and extracted with isopropyl ether (100 ml). The organic layer was separated, washed with water, dried ($Na_2SO_4$) and distilled to remove the solvent. The residue was

chromatographed on silica gel and elution was carried out with isopropyl ether and isopropyl ether-ethyl acetate (4:1) in the order mentioned. By the above procedure there was obtained the desired compound (II):

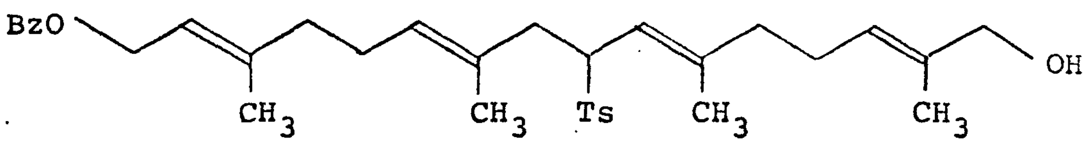

,

in an amount of 1.87 g, 68%. (See Table 1 below).

<u>Example I-17</u>

A sulfonyl compound (IV: Ts $-(Pr)_9$H, 360 mg, 0.47 m mole) and a bromide (III: Q-Pr-Br, 250 mg, 0.5 m mole) were dissolved in a mixture of THF (4 ml) and DMF (1 ml) and, under an argon gas atmosphere, the mixture was cooled to -5°C. To this mixture was added potassium tert-butoxide (0.1 g, 0.9 m mole). The mixture was stirred for 30 minutes, after which time it was diluted with water (20 ml) and extracted with isopropyl ether (50 ml). The product was isolated in the conventional manner and chromatographed on silica gel. Elution with isopropyl ether-hexane (1:1) yielded the desired compound (II):

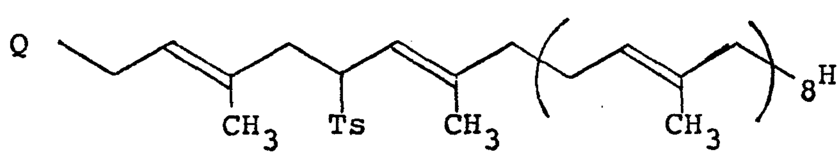

,

in an amount of 490 mg, 86%. (See Table 1 below).

<u>Example I-36</u>

A sulfonyl compound (IV: Ts $-(Pr)_4$H, 858 mg, 2 m moles) and a bromide (III:

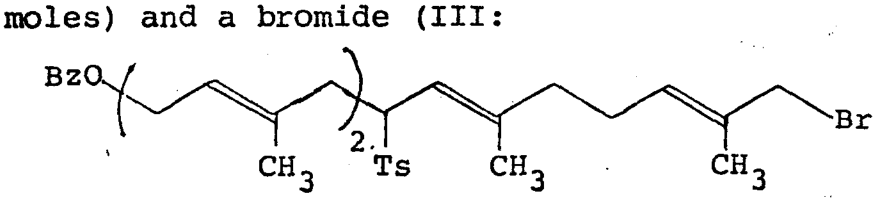

,

1.23 g, 2 m moles) were dissolved in DMF (20 ml) and the solution was cooled under an argon gas atmosphere. To this solution was added sodium hydride (50% oily dispersion, 200 mg, 4 m moles), and the mixture was stirred for 30 minutes. To this reaction mixture was added a further amount (100 mg, 2 m moles) of sodium hydride, followed by stirring for 30 minutes. The product was isolated and purified in conventional manner. By the above procedure there was obtained the desired compound (II):

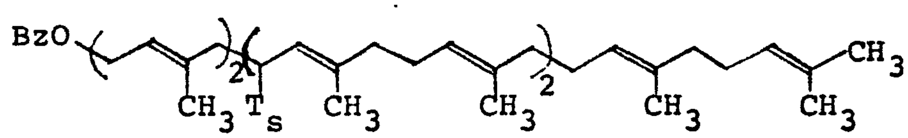 ,

in an amount of 1.65 g, 86%. (See Table 1 below).

<u>Example I-43</u>

A sulfonyl compound (IV;

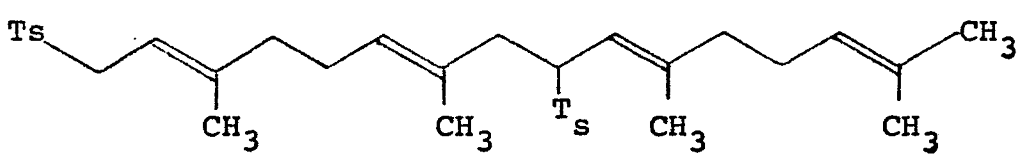 ,

560 mg, 1 m mole) and a bromide (III: BzO $\leftarrow$Pr$\rightarrow_2$Br, 323 mg, 1 m mole) were dissolved in DMF (10 ml) and, in an argon gas atmosphere, the solution was cooled to 5°C. To this solution was added sodium hydride (50% oil dispersion, 150 mg, 3 m moles) and the mixture was stirred for 60 minutes. The product was isolated and purified in the conventional manner. By the above procedure there was obtained the desired compound (see Table 1 below).

By procedures similar to those described in the foregoing examples, the starting compound (IV) and (III) given in Table 1 below were reacted under the conditions set forth in the same table to obtain the corresponding coupled compounds (II) (Table 1 includes the preceding examples) :

Table 1

$$R^1CH_2Y + XCH_2R^2 \longrightarrow R^1\overset{Y}{\overset{|}{C}}HCH_2R^2$$

(IV) (III) (II)

| Example I - | Starting Compounds (IV) (III) | °Basic Reagent<br>°Solvent | °Conditions of reaction<br>°Reaction time<br>°Yield | NMR spectrum of (II):<br>δ |
|---|---|---|---|---|
| 1 | Ts $\leftarrow Pr \rightarrow_9 H$<br>BzO-Pr-Br | t-BuOK<br>THF:DMF<br>(4:1) | -5°C, Ar<br>30 min<br>92% | 1.21(3H),<br>1.51(3H)<br>1.59(21H),<br>1.67(3H),<br>2.00 (32H),<br>2.41(3H),<br>2.84(1H),<br>3.94(2H),<br>4.46(2H),<br>4.9-5.2(9H),<br>5.35(1H),<br>7.27(5H),<br>7.26 & 7.71 (4H) |
| 2 | BzO-Pr-Ts<br>Br $\leftarrow Pr \rightarrow_9 H$ | t-BuOK<br>THF:DMF<br>(4:1) | 5°C, Ar<br>30 min.<br>87% | 1.60(27H),<br>1.68(6H),<br>1.8-2.2(32H),<br>2.41(3H),<br>2.7(2H),<br>3.45(1H),<br>3.95(2H),<br>4.38(2H),<br>4.9-5.2(9H),<br>5.33(1H),<br>7.26(5H),<br>7.26 & 7.71 (4H) |

| | | | | |
|---|---|---|---|---|
| 3 | Ts $(Pr)_8H$<br>BzO$(Pr)_2Cl$ | t-BuOK<br>THF:DMF<br>(4:1) | 5°C, Ar<br>30 min.<br>88% | 1.24(3H), 1.54 (3H), 1.62(24H), 1.68(3H), 2.23 & 2.87(2H), 2.44(3H), 3.86 (1H), 4.01(2H), 4.49(2H), 4.91 (1H), 5.0-5.2 (8H), 5.38(1H), 7.32(5H), 7.29 & 7.71(4H) |
| 4 | Ts$(Pr)_8H$<br>BzO$(Pr)_2Br$ | t-BuOK<br>THF:DMF<br>(4:1) | 5°C, Ar<br>30 min.<br>91% | do |
| 5 | Ts$(Pr)_2H$<br>BzO$(Pr)_2Br$ | NaH<br>DMF | 5°C, $N_2$<br>60 min.<br>90% | 1.24(3H), 1.54 (3H), 1.61(6H), 1.68(3H), 2.44 (3H), 2.23 & 2.87(2H), 3.86 (1H), 4.01(2H), 4.49(2H), 4.89 (1H),5.0-5.2 (2H), 5.37(1H), 7.28 & 7.71(4H), 7.33(5H) |
| 6 | Ts$(Pr)_2H$<br>BzO$(Pr)_2Cl$ | NaH<br>DMF | 20°C, $N_2$<br>60 min.<br>88% | do |
| 7 | Ts$(Pr)_3H$<br>BzO$(Pr)_2Br$ | t-BuOK<br>THF:DMF<br>(4:1) | 0°C, Air<br>30 min.<br>88% | 1.24(3H), 1.54 (3H), 1.61(9H), 1.59(3H), 2.23 & 2.87(2H), 2.44 (3H), 3.86(1H), 4.01(2H), 4.50 (2H), 4.90(1H), 4.95-5.24(3H), 5.37(1H), 7.30 & 7.72(4H), 7.33 (5H) |
| 8 | Ts$(Pr)_3H$<br>BzO$(Pr)_2Cl$ | NaH<br>DMF | 20°C, $N_2$<br>90 min.<br>89% | do |

| | | | | |
|---|---|---|---|---|
| 9 | $Ts(Pr)_2OH$<br>$BzO(Pr)_2Br$ | NaH<br>DMF | 5°C, Ar<br>90 min.<br>68% | 1.29(3H), 1.49 (3H), 1.59(3H), 1.64(3H), 2.17 & 2.73(2H), 2.31 (OH), 2.41(3H), 3.83(1H), 3.94 (2H), 3.97(2H), 4.46(2H), 4.86 (1H), 5.08(1H), 5.33(2H), 7.29 (5H), 7.2 & 7.7 (4H) |
| 10 | $BzO(Pr)_2Ts$<br>$Br(Pr)_5H$ | NaH<br>DMF | 20°C, Ar<br>90 min.<br>86% | 1.60(18H), 1.67 (3H), 1.68(3H), 1.8-2.2(20H), 2.40(3H), 3.42 (1H), 3.98(2H), 4.45(2H), 4.89 (1H),4.9-5.2(5H), 5.30(1H), 7.25 & 7.68(4H), 7.32 (5H) |
| 11 | $Ts(Pr)_3H$<br>$Br(Pr)_3H$ | NaH<br>DMF | 5°C, Ar<br>40 min.<br>92% | 1.25(3H), 1.54 (3H), 1.58(12H), 1.68(6H), 1.8-2.1(16H), 2.42 (3H), 3.74(1H), 4.8-5.3(6H),7.3 & 7.7(4H) |
| 12 | $Ts(Pr)_3H$<br>$Cl(Pr)_2H$ | NaH<br>DMF | 15°C, Ar<br>60 min.<br>90% | 1.25(3H), 1.54 (3H), 1.58(9H), 1.65(3H), 1.68 (3H), 2.42(3H), 3.75(1H), 4.8-5.3(5H), 7.3 & 7.7(4H) |
| 13 | $Ts(Pr)_2H$<br>$Br(Pr)_2H$ | NaH<br>DMF | 5°C, Ar<br>40 min.<br>98% | 1.23(3H), 1.57 (9H), 1.63(3H), 1.67(3H), 2.1-3.0(2H), 2.41 (3H), 3.68(1H), 4.9-5.1(4H), 7.25 & 7.69(4H) |

| | | | | |
|---|---|---|---|---|
| 14 | BzO(Pr)$_2$Ts<br>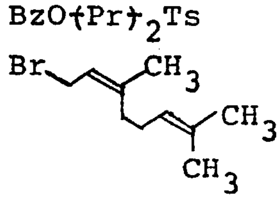 | NaH<br>DMF | 20°C, $N_2$<br>60 min<br>87% | 1.58(6H), 1.64(9H), 1.8-2.2(8H), 2.41 (3H), 2.73(2H), 3.40 (1H), 4.02(2H), 4.51 (2H), 4.91(1H), 4.9-5.3(2H), 5.36(1H), 7.31 & 7.71(4H), 7.36 (5H) |
| 15 | Ts(Pr)$_6$H<br>BzO(Pr)$_2$Cl | NaH<br>DMF | 20°C, $N_2$<br>60 min.<br>90% | 1.23(3H), 1.54(3H), 1.62(18H), 1.68(3H), 2.43(3H), 2.23 & 2.87 (2H), 3.86(1H), 4.01 (2H), 4.49(2H), 4.91 (1H), 5.0-5.2(6H), 5.37(1H), 7.32(5H), 7.29 & 7.71(4H) |
| 16 | 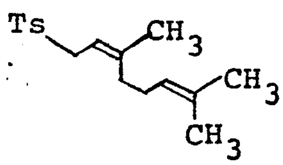<br>BzO(Pr)$_2$Cl | NaH<br>DMF | 5°C, $N_2$<br>120 min.<br>85% | 1.54(3H), 1.62(3H), 1.64(3H), 1.67(3H), 1.70(3H), 2.0-2.40 (8H), 2.44(3H), 3.80 (1H), 4.01(2H), 4.49 (2H), 4.80-5.30(3H), 5.40(1H), 7.29 & 7.74 (4H), 7.34(5H) |
| 17 | Ts(Pr)$_9$H<br>Q-Pr-Br | t-BuOK<br>THF:DMF<br>(4:1) | -5°C, Ar<br>30 min.<br>86% | 1.16(3H), 1.54(3H), 1.58(21H), 1.66(3H), 2.00(32H), 2.39(3H), 2.85(2H), 3.21(2H), 3.73(1H), 3.98(6H), 4.89(4H), 4.80(1H), 5.1(9H), 7.34(10H), 7.24 & 7.65(4H) |
| 18 | Ts(Pr)$_2$H<br>Q(Pr)$_2$Br | t-BuOK<br>THF:DMF<br>(4:1) | 0°C, Ar<br>30 min.<br>90% | 1.22(3H), 1.50(3H), 1.56(3H), 1.64(3H), 1.65(3H), 2.00(3H), 2.42(3H), 2.85(2H), 3.83(1H), 3.32(2H), 3.97(6H), 4.8-5.22 (4H), 4.97(4H), 7.2-7.7(14H) |

| | | | | |
|---|---|---|---|---|
| 19 | Q-Pr-Ts<br>$Br(Pr)_9H$ | t-BuOK<br>THF:DMF<br>(4:1) | 0°C, Ar<br>30 min.<br>88% | 1.52(3H), 1.60(24H), 1.70(6H), 1.90(3H), 2.00(32H), 2.30(3H), 3.26(3H), 3.34(1H), 3.87(3H), 3.92(3H), 4.7-5.2(10H), 4.84 (2H), 4.91(2H), 7.12 & 7.59(4H), 7.34(10H) |
| 20 | $Q(Pr)_2Ts$<br>$Br(Pr)_8H$ | t-BuOK<br>THF:DMF<br>(4:1) | 0°C, Ar<br>30 min.<br>86% | 1.52(3H), 1.60(24H), 1.68(6H), 2.10(3H), 2.41(3H), 2.72(2H), 3.43(1H), 3.31(2H), 3.97(6H), 4.78-5.20 (10H), 4.97(4H), 7.2-7.6(10H), 7.24 & 7.66 (4H) |
| 21 | $Ts(Pr)_3H$<br>$Q(Pr)_7Cl$ | NaH<br>DMF | 0°C, Ar<br>60 min.<br>82% | 1.24(3H), 1.51(3H), 1.60(21H), 1.68(6H), 2.00($-CH_2-$), 2.13(3H), 2.42(3H), 3.30 & 3.36 (2H), 3.84(1H), 3.93 & 3.94(6H), 5.1(10H), 7.28 & 7.72(4H), 7.3-7.6(10H) |
| 22 | $Ts(Pr)_2H$<br>Q-Pr-Br | NaH<br>DMF | 0°C, Ar<br>60 min.<br>83% | 1.15(3H), 1.53(3H), 1.57(3H), 1.63(3H), 1.7-2.0(4H), 1.99(3H), 2.39(3H), 2.6-2.95 (2H), 3.23(2H), 3.67-4.0(1H), 3.89(6H), 4.70-5.15(3H), 4.89 (4H), 7.2-7.64(14H) |
| 23 | $Q(Pr)_2Ts$<br>$Cl(Pr)_2H$ | NaH<br>DMF | 0°C, Ar<br>60 min.<br>85% | 1.5-2.1(15H), 2.09(3H), 2.38(3H), 2.70(2H), 3.42(1H), 3.30(2H), 3.97(6H), 4.78-5.20(4H), 4.97(4H), 7.20-7.60 (10H), 7.24 & 7.66(4H) |

| | | | | |
|---|---|---|---|---|
| 24 | Ts(Pr)$_2$H<br>BzBr | NaH<br>DMF | 5°C, Ar<br>60 min.<br>99% | 1.00(3H), 1.60(3H), 2.42(3H), 2.73 & 3.45 (2H), 3.93(1H), 3.93 (2H), 5.00(1H), 5.21 (1H), 7.13(5H), 7.28 & 7.73(4H) |
| 25 | Ts(Pr)$_2$H<br>Br(Pr)$_2$H | NaH<br>t-BuOH | 25°C, Ar<br>60 min.<br>77% | cf. Example I-13 |
| 26 | Ts(Pr)$_2$H<br>BzO(Pr)$_2$Br | t-BuONa<br>t-BuOH | 25°C, Ar<br>120 min.<br>68% | cf. Example I-5 |
| 27 | Ts(Pr)$_2$H<br>BzO(Pr)$_2$Br | $NaNH_2$<br>DMF | 5°C, Ar<br>60 min.<br>82% | cf. Example I-5 |
| 28 | MK(Pr)$_2$Ts<br>Cl(Pr)$_2$H | NaH<br>DMF | 5°C, Ar<br>60 min.<br>89% | 1.52(3H), 1.54(3H), 1.60(6H), 1.73(3H), 2.33(6H), 3.40(1H), 3.50(2H), 3.84(6H), 4.60-5.20(4H), 7.42 (2H), 7.61(2H), 7.30-7.50(2H), 7.9-8.15(2H) |
| 29 | E(Pr)$_2$Ts<br>Cl(Pr)$_2$H | NaH<br>DMF | 50°C, Ar<br>60 min.<br>90% | 1.56(3H), 1.59(3H), 1.63(3H), 1.72(3H), 1.8-2.2(6H), 2.20(9H), 2.40(3H), 2.5-2.7(2H), 3.33(2H), 3.40(1H), 3.65(3H), 3.66(3H), 4.90(1H), 4.8-5.15(3H), 7.24 & 7.65(4H) |
| 30 | Q-Pr-Ts<br>Br(Pr)$_2$H | NaH<br>DMF | 5°C, Ar<br>60 min.<br>84% | 1.51(6H), 1.67(3H), 1.71(3H), 1.90(3H), 2.29(3H), 2.4-2.7(2H), 3.26(2H), 3.34(1H), 3.87(3H), 3.92(3H), 4.7-5.15(3H), 4.84(2H), 4.91(2H), 7.12 & 7.59 (4H), 7.36(10H) |

| | | | | |
|---|---|---|---|---|
| 31 | 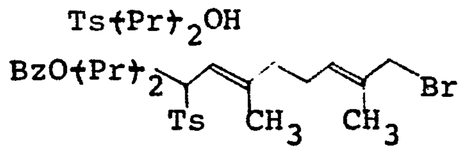 | NaH DMF | 5°C, Ar 90 min. 56% | 1.21(3H), 1.28 (3H), 1.47(6H), 1.58(3H), 1.63 (3H), 2.17 & 2.76(4H), 2.41 (6H), 3.81(2H), 3.92(2H), 3.95 (2H), 4.44(CH2), 4.84(2H), 5.0-5.2(2H), 5.30(2H), 7.28(5H), 7.2 & 7.7(4H) |
| 32 | 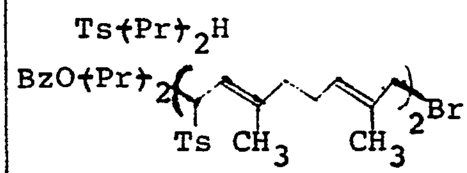 | NaH DMF | 5°C, Ar 60 min. 78% | 1.24(9H), 1.55 (9H), 1.61(6H), 1.71(3H), 1.8-2.1(16H), 2.44 (9H), 2.88(3H), 3.90(3H), 4.01 (2H), 4.50(2H), 4.90(3H), 5.0-5.4(5H), 7.31 (6H), 7.33(5H), 7.75(6H) |
| 33 | 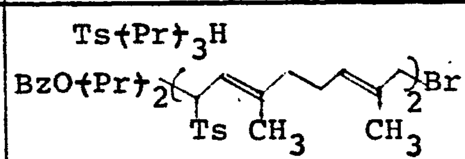 | NaH DMF | 5°C, Ar 60 min. 80% | 1.21(9H), 1.50 (9H), 1.58(9H), 1.67(3H), 2.19 & 2.82(6H),2.41 (9H), 3.82(3H), 3.97(2H), 4.46 (2H), 4.8-5.2 (8H), 4.33(1H), 7.30(5H), 7.25 & 7.67(12H) |
| 34 | 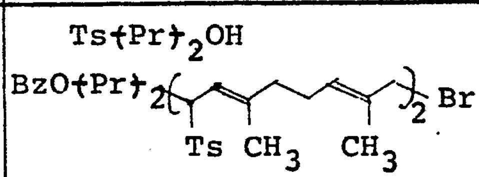 | NaH DMF | 5°C, Ar 60 min. 48% | 1.21(6H),1.28 (3H), 1.47(9H), 1.58(3H), 1.63 (3H), 2.17 & 2.76(6H), 2.41 (9H), 3.81(3H), 3.92(2H), 3.95 (2H), 4.44(2H), 4.84(3H), 5.0-5.2(3H), 5.30 (2H), 7.28(5H), 7.2-7.7(12H) |

| | | | | |
|---|---|---|---|---|
| 35 | $Ts(Pr)_2H$<br>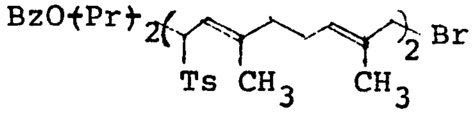 | NaH<br>DMF | 5°C, Ar<br>60 min.<br>72% | 1.24(12H),1.55(12H),1.61(6H),1.71(3H),1.8-2.1(20H),2.44(12H),2.88(4H),3.90(4H),4.01(2H),4.50(2H),4.90(4H),5.0-5.4(6H),7.31(8H),7.33(5H),7.73(8H) |
| 36 | $Ts(Pr)_4H$<br>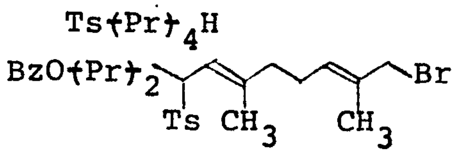 | NaH<br>DMF | 5°C, Ar<br>60 min.<br>86% | 1.21(6H),1.50(6H),1.58(12H),1.68(3H),2.41(6H),3.82(2H),3.97(2H),4.46(2H),4.9-5.2(7H),5.33(1H),7.30(5H),7.25 & 7.67(8H) |
| 37 | $Ts(Pr)_5H$<br>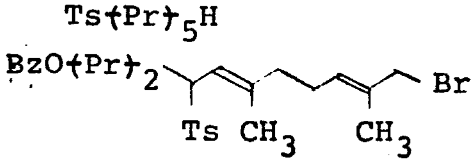 | NaH<br>DMF | 5°C, Ar<br>60 min.<br>88% | 1.22(6H),1.51(6H),1.59(15H),1.68(3H),2.42(6H),3.83(2H),3.97(2H),4.46(2H),4.9-5.2(8H),5.33(1H),7.30(5H),7.25 & 7.67(8H) |
| 38 | $Ts(Pr)_9H$<br>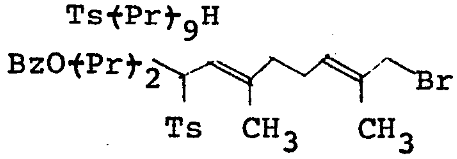 | t-BuOK<br>THF:DMF | 5°C, Ar<br>40 min.<br>86% | 1.21(6H),1.50(6H),1.59(27H),1.67(3H),2.41(6H),3.82(2H),3.97(2H),4.46(2H),4.9-5.2(12H),5.33(1H),7.30(5H),7.25 & 7.67(8H) |
| 39 | $Ts(Pr)_4H$<br>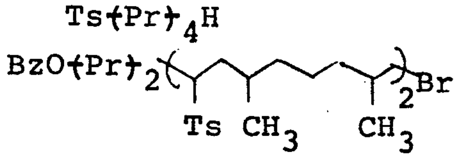 | NaH<br>DMF | 5°C, Ar<br>40 min.<br>89% | 1.22(9H),1.52(9H),1,59(12H),1.68(3H),2.41(9H),3.82(3H),3.97(2H),4.46(2H),4.9-5.2(9H),5.33(1H),7.30(5H),7.25 & 7.67(12H) |

| | | | | |
|---|---|---|---|---|
| 40 | $Ts\{Pr\}_8H$ 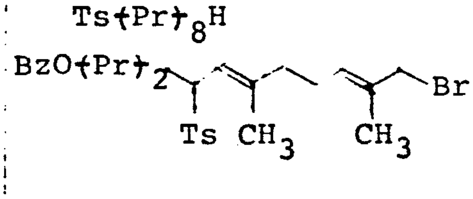 | t-BuOK THF: DMF | 5°C, Ar 40 min. 91% | 1.21(6H),1.50 (6H),1.58(24H), 1.68(3H),2.42 (6H),3.83(2H), 3.97(2H),4.46 (2H),4.9-5.2 (11H),5.33(1H), 7.30(5H),7.25 & 7.67(8H) |
| 41 | $Ts\{Pr\}_{12}H$ 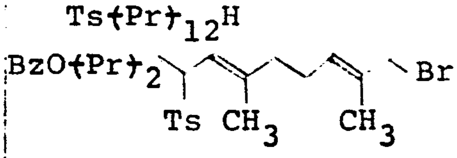 | t-BuOK THF: DMF | 5°C, Ar 40 min. 87% | 1.22(6H),1.51 (6H),1.58(36H), 1.68(3H),2.42 (6H),3.83(2H), 3.97(2H),4.46 (2H),4.9-5.2 (15H),5.33(1H), 7.30(5H),7.26 & 7.68(8H) |
| 42 | $Ts\{Pr\}_{16}H$ 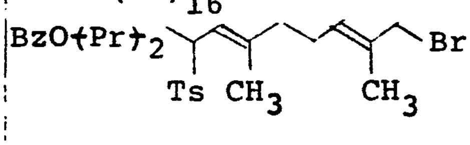 | t-BuOK THF: DMF | 5°C, Ar 60 min. 90% | 1.22(6H),1.51 (6H),1.58(48H), 1.68(3H),2.42 (6H),3.83(2H), 3.97(2H),4.46 (2H),4.9-5.2 (19H),5.33(1H), 7.30(5H),7.26 & 7.68(8H) |
| 43 | 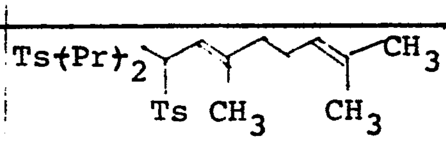 $BzO\{Pr\}_2Br$ | NaH DMF | 5°C, Ar 60 min. 82% | 1.24(6H),1.55 (6H),1.61(6H), 1.71(3H),1.8-2.1(12H),2.44 (6H),2.88(4H), 3.90(2H),4.01 (2H),4.50(2H), 4.90(2H),5.0-5.4(4H),7.31 (4H),7.33(5H), 7.74(4H) |
| 44 | 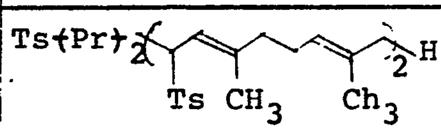 $BzO\{Pr\}_2Br$ | NaH DMF | 5°C, Ar 60 min. 78% | cf. Example I-32 |

## Example II

### Example II-1

Under an argon atmosphere, ethylamine (100 ml) was cooled to 0°C and, under stirring, lithium (2.80 g) was added. The mixture was stirred for 20 minutes, whereby a deep-blue solution was obtained. This solution was cooled to -35°C and a solution of the coupled compound (II :

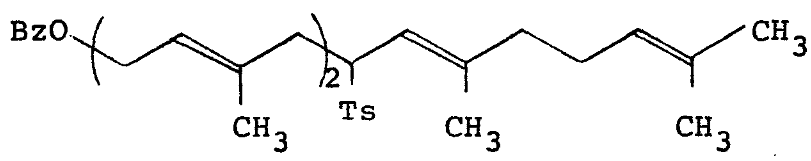

,

5.35 g, 10.0 m moles) in THF (25 ml) was added dropwise. After the dropwise addition was complete, the reaction was carried out under the same conditions for an additional 10 minutes, at the end of which time the excess anion radical was quenched by the addition of isoprene (2 ml). To this reaction mixture were added methanol (40 ml) and water (70 ml). The mixture was then concentrated under reduced pressure and the residue was extracted with isopropyl ether. The organic layer was washed, dried ($Na_2SO_4$) and concentrated. The residue was chromatographed on silica gel and developed with hexane-isopropyl ether (1:1). By the above procedure there was obtained the desired compound (I:$HO{-}(Pr)_4H$, 2.56 g, 88.3%). (Table 2 below)

### Example II-16

Under an argon atmosphere, ethylamine (20 ml) was cooled to -20°C, followed by the addition of lithium (300 mg) and the coupled compound (II:

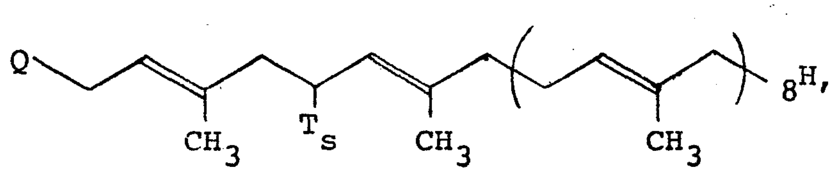

1.20 g, 1 m mole). The mixture was stirred for 30 minutes, whereupon it began to assume a blue colour. This blue solution was stirred for an additional 10 minutes. Thereafter, the excess radical anion was quenched by the addition of isoprene (0.5 ml). The ethylamine was removed under reduced pressure and the residue was added to deaerated cold THF (30 ml) containing 15% of acetic acid, care being exercised to avoid exposure to air. To this solution was added 10% aqueous ferric chloride (20 ml), followed by stirring for one hour. The reaction mixture was concentrated under reduced pressure and the concentrate was extracted with hexane. The organic layer was washed with water, dried and concentrated, whereby a crude product was obtained. This product was chromatographed on silica gel and developed with hexane-isopropyl ether (2:1). By the above procedure there was obtained ubiquinone-10 (630 mg) (Table 2 below). In IR and NMR spectra, this product was found to be identical with the ubiquinone-10 obtained from bovine heart.

Example II-20

Under an argon atmosphere, ethylamine (20 ml) was cooled to -20°C. Then, lithium (300 mg) and the coupled compound (II:

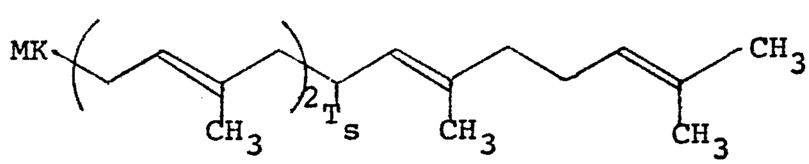

,

661 mg, 1 m mole) were added. The reaction was conducted until a blue solution was obtained. The solvent was removed under reduced pressure and the residue was extracted by the addition of hexane (50 ml) and water (20 ml). The product was isolated in the conventional

manner. By the above procedure there was obtained a dihydronaphthalene derivative ( 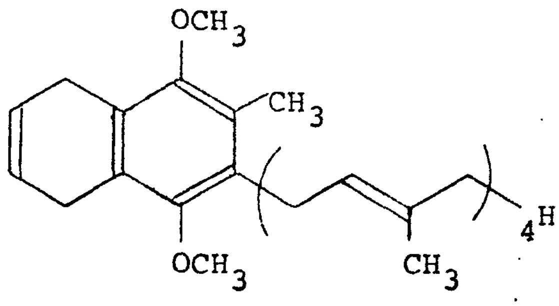 , 410 mg). This derivative was dehydrogenated in benzene (20 ml) using 2,3-dichloro-4,5-dicyano-1,4-benzoquinone (230 mg). The resulting product was isolated in conventional manner. By this procedure there was obtained menaquinol-4 dimethyl ether (403 mg). This dimethyl ether compound (403 mg) was dissolved in a mixture of dioxane (20 ml) and ether (20 ml) and the solution was cooled to -15°C. To this solution was added silver oxide (AgO, 800 mg), followed by the addition of 4N-nitric acid (0.8 ml). The mixture was stirred at -10°C for 10 minutes. To this was added hexane (100 ml) and the organic layer was washed with water, dried ($Na_2SO_4$) and concentrated under reduced pressure. The residue was chromatographed on silica gel and eluted with benzene. By the above procedure there was obtained menaquinone-4 (280 mg) (see Table 2).

By procedures similar to those described in the foregoing examples, the corresponding coupled compounds (II) were desulfonylated to the contemplated compounds (I) given below in Table 2 below. (Table 2 includes the preceding examples as well) :

Table 2

$$R^1\overset{Y}{C}HCH_2R^2 \longrightarrow R^1CH_2CH_2R^2$$

(II) (I)

| Example II- | (I) | Molecular formula | NMR spectrum (δ) |
|---|---|---|---|
| 1 | $HO(Pr)_4H$ | $C_{20}H_{34}O$ | 1.62(9H), 1.70(6H), 4.15(2H), 5.0-5.2(3H), 5.43(1H) |
| 2 | $HO(Pr)_5H$ | $C_{25}H_{42}O$ | 1.62(12H), 1.70(6H), 4.14(2H), 5.0-5.2(4H), 5.42(1H) |
| 3 | $HO(Pr)_6H$ | $C_{30}H_{50}O$ | 1.62(15H), 1.68(6H), 4.14(2H), 5.0-5.2(5H), 5.42(1H) |
| 4 | $HO(Pr)_7H$ | $C_{35}H_{58}O$ | 1.61(18H), 1.68(6H), 4.14(2H), 5.0-5.2(6H), 5.42(1H) |
| 5 | $HO(Pr)_8H$ | $C_{40}H_{66}O$ | 1.61(21H), 1.68(6H), 4.14(2H), 5.0-5.2(7H), 5.42(1H) |
| 6 | $HO(Pr)_9H$ | $C_{45}H_{74}O$ | 1.61(24H), 1.68(6H), 4.14(2H), 5.0-5.2(8H), 5.42(1H) |
| 7 | $HO(Pr)_{10}H$ | $C_{50}H_{82}O$ | 1.62(27H), 1.69(6H), 4.15(2H), 5.0-5.2(9H), 5.43(1H) |
| 8 | $HO(Pr)_{12}H$ | $C_{60}H_{98}O$ | 1.61(33H), 1.69(6H), 4.14(2H), 5.0-5.2(11H), 5.42(1H) |
| 9 | $HO(Pr)_{13}H$ | $C_{65}H_{106}O$ | 1.61(36H), 1.68([illegible]H), 4.14(2H), 5.0-5.[illegible](11H), 5.42(1H) |
| 10 | $HO(Pr)_{16}H$ | $C_{80}H_{130}O$ | 1.61(45H), 1.68([illegible]H), 4.14(2H), 5.0-5.[illegible] (12H), 5.42(1H) |
| 11 | $HO(Pr)_{20}H$ | $C_{100}H_{162}O$ | 1.61(57H), 1.68([illegible]H), 4.14(2H), 5.0-5.[illegible] (19H), 5.42(1H) |

0001873

| | | | |
|---|---|---|---|
| 12 | 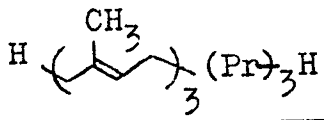 | $C_{30}H_{50}$ | 1.60(18H),1.67(6H), 5.09(6H) |
| 13 | 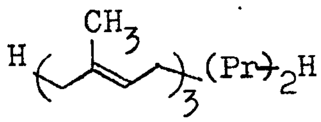 | $C_{25}H_{42}$ | 1.60(15H),1.67(6H), 5.1(5H) |
| 14 | 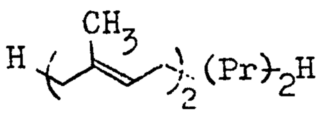 | $C_{20}H_{34}$ | 1.61(12H),1.68(6H), 5.1(4H) |
| 15 | 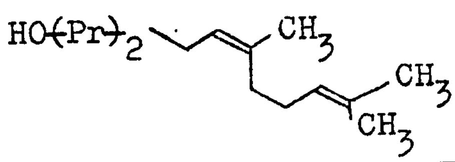 | $C_{20}H_{34}O$ | 1.62(6H),1.77(9H), 1.8-2.3(12H),4.18 (2H),5.0-5.36(3H), 5.47(1H) |
| 16 | Q'(Pr)$_{10}$H | $C_{59}H_{90}O_4$ | 1.61(27H),1.68(3H), 1.74(3H),2.02(36H), 3.18(2H),3.97(3H), 3.99(3H),5.1(10H) |
| 17 | Q'(Pr)$_4$H | $C_{29}H_{42}O_4$ | 1.61(9H),1.68(3H), 1.74(3H),3.18(2H), 3.97(3H),3.99(3H), 5.1(4H) |
| 18 | Q'(Pr)$_3$H | $C_{24}H_{34}O_4$ | 1.61(6H),1.68(3H), 1.74(3H),3.18(2H), 3.97(3H),3.99(3H), 5.1(3H) |
| 19 | Bz(Pr)$_2$H | $C_{17}H_{24}$ | 1.60(6H),1.67(3H), 7.18(5H) |
| 20 | MK'(Pr)$_4$H | $C_{31}H_{40}O_2$ | 1.68(9H),1.77(3H), 1.89(3H),1.98-2.15 ($CH_2$),2.20(3H),3.37 (2H),5.05(4H),7.6-7.75(2H),8.02-8.15 (2H) |
| 21 | E'(Pr)$_4$H | $C_{29}H_{42}O_2$ | 1.52(3H),1.58(9H), 1.68(3H),1.96(9H), 3.12(2H),4.8-5.1(4H) |
| 22 | HO(Pr)$_6$OH | $C_{30}H_{50}O_2$ | 1.61(12H),1.65(3H), 1.68(3H),3.92(2H), 4.14(2H),5.0-5.2 (4H),5.4(2H) |
| 23 | HO(Pr)$_8$OH | $C_{40}H_{66}O_2$ | 1.61(18H),1.65(3H), 1.68(3H),3.92(2H), 4.14(2H),5.0-5.2(6H), 5.4(2H) |

Reference Example 1

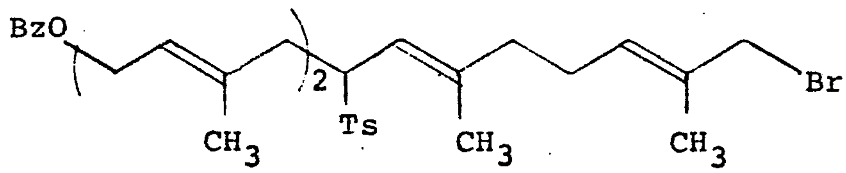

In dichloromethane (40 ml) was dissolved

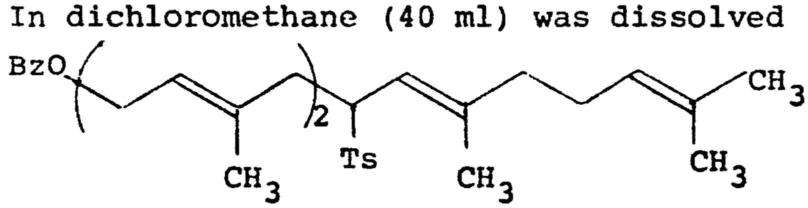

(5.34 g, 10 m moles) and the solution was cooled to -70°C. Then, a solution of benzene sulfenyl chloride (1.52 g, 10.5 m moles) in dichloromethane (10 ml) was added gradually to the above solution. After the dropwise addition was complete, the mixture was allowed to warm to room temperature and the solvent was distilled off. To the residue were added DMF (25 ml) and triethylamine (2.8 ml). The mixture was then stirred under heating at 80°C for 2 hours. After the reaction mixture was cooled, water (50 ml) and isopropyl ether (100 ml) were added to extract the product. The organic layer was washed with 3% aqueous phosphoric acid and water, dried ($Na_2SO_4$) and distilled to remove the solvent. The residue was subjected to silica gel chromatography. By the above procedure there was obtained a sulfide:

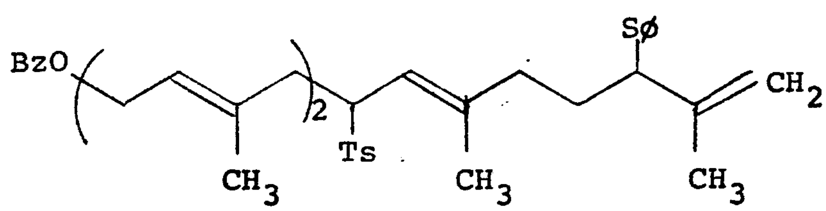

[5.69 g, 88.6%, δ1.20(3H), 1.50 (3H), 1.59(3H), 1.74(3H), 2.20 & 2.82(2H),

2.40(3H), 3.49(1H), 3.82(1H), 3.97(2H), 4.46(2H), 4.64(2H), 4.64(2H), 4.88(1H), 5.10(1H), 5.34(1H), 7.1-7.7(9H)]. This allylsulfide (5.50 g, 8.56 m moles) was dissolved in dichloromethane (50 ml) and the solution was cooled to -30°C.

To this solution was added metachloroperbenzoic acid (purity 80%, 1.85 g) under stirring over a period of 30 minutes. After the addition, the solution was further stirred under the same conditions for 10 minutes. The reaction mixture was allowed to warm to room temperature and the precipitated metachlorobenzoic acid was removed. The crystals were washed with dichloromethane and the washings were combined with the mother liquor. The dichloromethane solution was washed with aqueous sodium bicarbonate and water, dried ($Na_2SO_4$) and concentrated under reduced pressure. The residue was dissolved in methanol (50 ml). Trimethyl phosphite (3.2 g) was added and the mixture was allowed to stand at 24-26°C for 30 hours. The reaction mixture was concentrated under reduced pressure and the residue was chromatographed on silica gel, elution with isopropyl ether-ethyl acetate (9:1) yielding an alcohol of the formula:

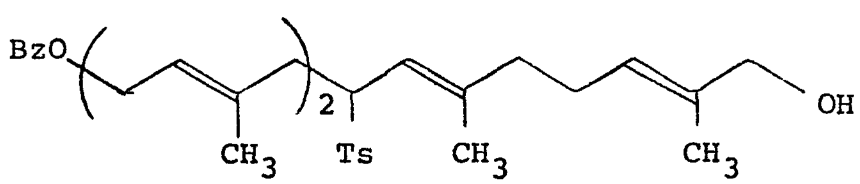

(3.97 g, 84.2%). This alcohol (2.2 g) was dissolved in isopropyl ether (20 ml) and cooled to 0°C. To this solution was added isopropyl ether (5 ml) containing phosphorus tribromide (1.0 g) under stirring. After the dropwise addition was complete, the mixture was stirred for 10 minutes. To the reaction mixture ice

(10 g) and, then, water were added. The organic layer was washed with aqueous sodium bicarbonate and water, dried and concentrated. By the above procedure there was obtained the captioned contemplated compound [2.1 g, 86%, δ 1.24(3H), 1.50(3H), 1.59(3H), 1.71(3H), 2.19-2.81(2H), 2.41(3H), 3.82(1H), 3.89(2H), 3.97(2H), 4.45 (2H), 4.87(1H), 5.0-5.5(3H), 7.29(5H), 7.25 & 7.67(4H)].

Reference Example 2

trans-2,5-Dibenzyloxy-3,4-dimethoxy-1-methyl-6-(3'-methyl-4'-p-toluenesulfonyl-2'-butenyl)-benzene (IV: Q-Pr-Ts).

In methylene chloride (150 ml) were dissolved 2,5-dibenzyloxy-3,4-dimethoxy-1-methyl-6-(2'-hydroxy-3'-methyl-3'-butenyl)-benzene (14 g) and triethylamine (10 g). The mixed solution was cooled to -40°C under a nitrogen stream, and a methylene chloride solution (20 ml) containing p-toluenesulfenyl chloride (7.2 g) was gradually added. After the addition, the mixture was stirred for 20 minutes at -30°C, and aqueous sodium bicarbonate was added. The reaction mixture was washed with water and dried ($Na_2SO_4$), and the solvent was evaporated. The residue was dissolved in methylene chloride, and to the solution were added sodium acetate (1 g) and 40% peracetic acid (8 g). The mixture was stirred at room temperature for 4 hours. After the reaction was complete, sodium carbonate and water were added, and the mixture was washed with water, dried and concentrated under reduced pressure. The residue was crystallized from isopropyl ether to give the captioned compound.

Yield: 16 g, m.p. 105-106°C.

δ 1.78(3H), 1.92(3H), 2.31(3H), 3.32(2H), 3.60(2H), 3.87(3H), 3.92(3H), 4.85(1H), 4.86(2H), 4.91(2H), 7.36(10H), 7.14(2H), 7.60(2H).

CLAIMS:

1. A method of producing a polyisoprenoid compound by carbon-carbon bond formation characterized in that a sulfonyl compound of the formula :

$$R^1CH_2Y \qquad (IV)$$

wherein Y is lower alkylsulfonyl or arylsulfonyl, and $R^1$ is isoprenoid or polyisoprenoid,

is reacted with a halogeno-compound of the formula:

$$XCH_2R^2 \qquad (III)$$

wherein X is halogen and $R^2$ has the same meaning as $R^1$,

in a polar solvent and in the presence of sodium hydride, potassium hydride, sodium tertiary-alkoxide, potassium tertiary-alkoxide, sodium amide or potassium amide, to obtain a compound of the formula:

$$R^1\overset{Y}{\overset{|}{C}}HCH_2R^2 \qquad (II)$$

wherein Y, $R^1$ and $R^2$ have the same meanings as defined above,

which, in turn, is subjected to a reductive desulfonylation of the group Y, whereby a compound of the formula:

$$R^1CH_2CH_2R^2 \qquad (I)$$

wherein $R^1$ and $R^2$ have the same meanings as defined above,

is obtained.

2. The method according to claim 1, further characterized in that the reaction of a sulfonyl compound with a halogeno-compound is conducted in a polar solvent and in the presence of sodium hydride, potassium

hydride, sodium tertiary-$C_{4-6}$ alkoxide, potassium tertiary-$C_{4-6}$ alkoxide, sodium amide or potassium amide.

3. The method according to claim 2, further characterized in that the polar solvent is dimethylformamide, dimethylacetamide, tertiarybutyl alcohol, tertiary-amyl alcohol, dimethylsulfoxide, tetrahydrofuran or 1,2-dimethoxyethane.

4. The method according to any of claims 1 to 3, wherein Y is arylsulfonyl.

5. The method according to claim 4, wherein the arylsulfonyl is p-toluenesulfonyl.

6. The method according to any of claims 1 to 5, wherein the halogen X is chlorine or bromine.

7. The method according to any of claims 1-6, wherein the polyisoprenoid compound to be produced is ubiquinone-10.

8. The method according to any of claims 1-6, wherein the polyisoprenoid compound to be produced is all trans-tetraprenyl alcohol.

9. The method according to any of claims 1-6, wherein the polyisoprenoid compound to be produced is all trans-decaprenyl alcohol.

0001873

European Patent Office

# EUROPEAN SEARCH REPORT

Application number
EP 78 300 205.8

## DOCUMENTS CONSIDERED TO BE RELEVANT

| Category | Citation of document with indication, where appropriate, of relevant passages | Relevant to claim |
|---|---|---|
| X | GB - A - 1 396 627 (RHONE-POULENC S.A.) | 1 |
| | * page 3, lines 53-54; | |
| | page 4, lines 11-19; | |
| | page 4, lines 34-35; | |
| | page 4, lines 7-10; | 2,3 |
| | page 4, lines 25-27; | |
| | page 1, lines 8- 9; | 4 |
| | page 2, line 57 * | 6 |
| | -- | |
| - | US - A - 3 804 882 (M.JULIA) | 1 |
| | * page 1, lines 15-45 * | |
| | -- | |
| A | DE - B - 1 642 646 (NISSHIN FLOUR MILLING) | 7 |
| | * column 1, line 5 * | |
| | ---- | |

CLASSIFICATION OF THE APPLICATION (Int. Cl.²)

C 07 C 11-21
C 07 C 33-02
C 07 C 49-73

TECHNICAL FIELDS SEARCHED (Int.Cl.²)

C 07 C 11-21
C 07 C 33-02
C 07 C 49-73
C 07 C 147-04

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 08-11-1978 | KNAACK |